# EUROPEAN PATENT APPLICATION

(11) **EP 3 332 787 A1**
(43) Date of publication of application: **13.06.2018**
(21) Application number: 16851912.2
(22) Date of filing: 30.09.2016
(51) Int. Cl.: A61K 31/568, A61K 47/10, A61K 47/32, A61K 47/34, A61P 5/24

(54) **TRANSDERMALLY ABSORBABLE COMPOSITION**

(30) Priority: 30.09.2015 JP 2015194354; 14.07.2016 JP 2016139690
(71) Applicant: Fujifilm Corporation, Minato-ku Tokyo 106-8620 (JP)
(72) Inventor: SONOKE, Shirou, Ashigarakami-gun Kanagawa 258-8577 (JP); IZUMI, Yasuyuki, Ashigarakami-gun Kanagawa 258-8577 (JP); YAMAZAKI, Naomi, Ashigarakami-gun Kanagawa 258-8577 (JP)
(74) Representative: Hoffmann Eitle
(86) International application number: PCT/JP2016/079149
(87) International publication number: WO 2017/057743

(57) **Abstract**

Provided is a composition for percutaneous absorption containing testosterone and one or more organic solvents selected from the group consisting of propylene glycol, 1,3-butylene glycol, dipropylene glycol and polyethylene glycol, in which a total content of the organic solvents with respect to a total amount of the composition for percutaneous absorption is from 60% by mass to 90% by mass, and in which a content of a monohydric alcohol having 2 to 4 carbon atoms with respect to the total amount of the composition for percutaneous absorption is equal to or less than 10% by mass.

## Description

### BACKGROUND OF THE INVENTION

### 1. Field of the Invention

The present invention relates to a composition for percutaneous absorption.

### 2. Description of the Related Art

Male sexual dysfunction results from various physiological phenomena such as aging or a disease-induced decrease in testosterone levels below a normal range. Particularly, testosterone decreases due to aging. Among the testosterones in the body, the testosterone binding to high-affinity sex hormone-binding globulin (hereinafter, referred to as SHBG in some cases) is difficult to use physiologically. The level of SHBG in the blood tends to increase with age. Therefore, among the testosterones in the body, the testosterone that can be physiologically used tends to further decrease with age.

In addition to aging, general diseases such as renal failure and diabetes, disorders such as hypogenitalism, physiological conditions such as obesity can cause a decrease in testosterone levels in some cases.

The sexual dysfunction described above is likely to lead to various physiological changes including, for example, a decrease in sex drive, impotence, a reduction in muscle mass, a reduction in bone density, depression, a decrease in strength, and a cognitive decline. As a result, senile sexual dysfunction which is a disease so-called "andropause" develops in some cases.

For treating the disease, a male hormone such as testosterone is used, and the male hormone such as testosterone is administered through hormone replacement therapy by using oral agents, injections, percutaneous absorption preparations, and the like.

In the hormone replacement therapy, in a case where a natural hormone biosynthesized in a biological body is orally administered, because the hormone is poorly absorbed from the gastrointestinal tract but is extremely rapidly metabolized in the liver, the hormone needs to be administered multiple times at a high dose. Therefore, a burden is laid on the patient. In addition, the administration of the hormone by using injections also lays a big burden on the patient.

It is important to administer testosterone into the body at an effective dose. Therefore, for the purpose of avoiding at least the initial metabolism in the liver at the time of administering testosterone into the biological body, various methods for administering testosterone by using a composition for percutaneous absorption are being attempted, and it is desired that a composition for percutaneous absorption which enables testosterone to efficiently permeate the skin needs to be provided.

Generally, in the process in which a drug from a composition for percutaneous absorption permeates the skin, in order to increase the permeation rate of the drug, it is necessary to assure that the drug exhibits sufficient solubility in at least the composition.

For example, a method is suggested which is for treating the sexual dysfunction of a male subject by using a testosterone-containing composition having excellent percutaneous absorptivity by the dermal administration of a pharmaceutical composition in the dosage form of an aqueous alcoholic gel containing testosterone, isopropyl myristate, 60% to 80% of an alcohol selected from the group consisting of ethanol and isopropanol, a thickener, and water (for example, see JP2009-511602A).

Furthermore, a percutaneous pharmaceutical preparation is suggested which contains a testosterone-containing active medication and a mixture of (ii) glycol, (iii) alcohol having 2 to 4 carbon atoms, and water as a solvent system (for example, see JP2007-508261A). In addition, JP2007-508261A describes that (iv) mixture of the lower alcohol and water, which is contained in the aforementioned percutaneous pharmaceutical preparation and presents in an amount of about 40% to 98% of the solvent system, can contain the lower alcohol in an amount of about 5% to 80% and the water in an amount of about 20% to 95%.

As another example of a composition for percutaneous absorption, a single-phase gel-like composition is suggested which contains a testosterone-containing active medication, a polymer being able to form a gel, and a gelling agent and can impart absorptivity to the active medication (for example, see US2006/029223).

### SUMMARY OF THE INVENTION

In the composition used in the method described in JP2009-511602A, a large amount of lower alcohol is used for solubilizing the active medication. Therefore, there is a concern about the skin irritation caused by the lower alcohol. In the preparation described in JP2007-508261A, in addition to glycol, a mixture of an alcohol and water is used as a solvent. Furthermore, JP2007-508261A describes that the content of the alcohol having 2 to 4 carbon atoms can be in a range of 5% by mass to 80% by mass in the mixture. However, in JP2007-508261A, as a formulation example of the preparation, only an example in which the content of the alcohol is equal to or greater than 30% by mass is disclosed. Therefore, the problem of a skin irritation resulting from the lower alcohol contained in the preparation still exists.

The gel-like composition described in US2006/029223 is a gel-like composition containing a polymer which can form a gel represented by propylene glycol and a carbomer (a carboxyvinyl polymer) which is a gelling agent. US2006/029223 discloses an example in which testosterone is contained in the composition in an amount of 0.015% by mass. Although the gel-like composition described in US2006/029223 does not contain a lower alcohol, it is a gel-like composition not containing water. Therefore, it is difficult to efficiently manufacture a uniform composition, and in view of manufacturing properties, the composition needs to be further improved.

US2006/029223 describes an example in which a composition containing 0.015% by mass of testosterone, 97.485% by mass of propylene glycol, and 2.5% by mass of carboxyvinyl polymer is prepared by stirring at a high shear rate (for example, 50 Pa to 250 Pa). Unfortunately, the stirring device of a high shear rate is expensive. Furthermore, according to the examination performed by the inventors of the present invention, it was found that in a case where the aforementioned composition is prepared by stirring by using the stirring device of a high shear rate, unfortunately, the gelling agent such as a carboxyvinyl polymer is decomposed.

Although various examinations have been conducted as described above, a composition for percutaneous absorption just as a testosterone-containing percutaneous absorption preparation, in which testosterone is sufficiently solubilized and exhibits excellent skin permeability and which is inhibited from causing skin irritation, has not yet been obtained.

An object of an embodiment of the present invention is to provide a composition for percutaneous absorption which has excellent skin permeability of testosterone and is inhibited from causing skin irritation.

The present invention includes the following embodiments.
[1] A composition for percutaneous absorption, comprising testosterone and one or more organic solvents selected from the group consisting of propylene glycol, 1,3-butylene glycol, dipropylene glycol and polyethylene glycol, in which a total content of the organic solvents with respect to a total amount of the composition for percutaneous absorption is 60% by mass to 90% by mass, and a content of a monohydric alcohol having 2 to 4 carbon atoms with respect to the total amount of the composition for percutaneous absorption is equal to or less than 10% by mass.
[2] The composition for percutaneous absorption described in [1], further comprising water, in which a content of the water with respect to the total amount of the composition for percutaneous absorption is 5% by mass to 35% by mass.
[3] The composition for percutaneous absorption described in [1] or [2], in which the content of the monohydric alcohol having 2 to 4 carbon atoms with respect to the total amount of the composition for percutaneous absorption is equal to or less than 4% by mass.
[4] The composition for percutaneous absorption described in any one of [1] to [3], in which the content of the monohydric alcohol having 2 to 4 carbon atoms with respect to the total amount of the composition for percutaneous absorption is equal to or less than 0.1% by mass.
[5] The composition for percutaneous absorption described in any one of [1] to [4], in which a content of testosterone with respect to the total amount of the composition for percutaneous absorption is from 0.5% by mass to 10% by mass.
[6] The composition for percutaneous absorption described in any one of [1] to [5], in which the organic solvents include at least one or more organic solvents selected from the group consisting of propylene glycol and 1,3-butylene glycol.
[7] The composition for percutaneous absorption described in any one of [1] to [6], in which the organic solvents include propylene glycol.
[8] The composition for percutaneous absorption described in any one of [1] to [7], further comprising a gelling agent.
[9] The composition for percutaneous absorption described in any one of [1] to [8], in which a viscosity measured at a shear rate of 100 s⁻¹ at 25°C is within a range of 0.5 Pa·s to 5 Pa·s.

According to an embodiment of the present invention, it is possible to provide a composition for percutaneous absorption which has excellent skin permeability of testosterone and is inhibited from causing skin irritation.

### DESCRIPTION OF THE PREFERRED EMBODIMENTS

The composition for percutaneous absorption of the present disclosure (hereinafter, simply referred to as a composition in some cases) contains testosterone and one or more organic solvents (hereinafter, referred to as "specific organic solvents" in some cases) selected from the group consisting of propylene glycol (PG), 1,3-butylene glycol (BG), dipropylene glycol (DPG), and polyethylene glycol (PEG), in which a total content of the specific organic solvents with respect to a total amount of the composition for percutaneous absorption is 60% by mass to 90% by mass, and a content of a monohydric alcohol having 2 to 4 carbon atoms with respect to the total amount of the composition for percutaneous absorption is equal to or less than 10% by mass.

Hereinbelow, in some cases, propylene glycol will be referred to as PG, 1,3-butylene glycol will be referred to as BG, dipropylene glycol will be referred to as DPG, and polyethylene glycol will be referred to as PEG.

The composition of the present disclosure contains the specific organic solvents selected from the group consisting of the aforementioned hydrophilic organic solvents in the specific amount described above. Therefore, the composition has excellent solubility and skin permeability of testosterone. Presumably, for this reason, even in a case where the composition does not contain a monohydric alcohol having 2 to 4 carbon atoms that is generally used as a solvent excellently dissolving testosterone or even in a case where the composition contains such a monohydric alcohol in an extremely small amount, the skin permeability of testosterone becomes excellent. Accordingly, the composition of the present disclosure will not cause skin irritation resulting from the monohydric alcohol having 2 to 4 carbon atoms, can be applied to any region of the skin, and is expected to make it possible to obtain the efficacy of testosterone.

Furthermore, the composition contains the specific hydrophilic organic solvent in the specific amount described above. Therefore, in a case where the composition is applied to the skin, the composition feels good on the skin. In addition, the composition has an affinity for the skin and spreads well at the time of application. Accordingly, the composition has advantages that it is excellently retained on the skin by being applied to a desired region of the skin, can be easily manufactured using a widely used stirring device, and has excellent manufacturing properties.

Herein, the present disclosure is not limited to the presumptive mechanism described above.

In the present specification, a range of numerical values described using "to" is a range including the numerical values listed before and after "to" as a minimum value and a maximum value respectively.

In the present specification, the upper limit or the lower limit of a range of numerical values that is gradationally described may be substituted with the upper limit or the lower limit of another range of numerical values that is gradationally described. Furthermore, in the present specification, the upper limit or the lower limit of a range of numerical values may be substituted with the values shown in examples.

In addition, in the present specification, in a case where the composition contains a plurality of substances as each component, unless otherwise specified, the amount of each component contained in the composition means the total amount of the plurality of substances.

Hereinafter, each component contained in the composition of the present disclosure will be specifically described.

### [Testosterone]

The composition for percutaneous absorption contains testosterone as an active medication.

Testosterone which is a major circulating androgen in a male is synthesized from cholesterol. Through two different pathways, the circulating testosterone is metabolized into 17-ketosteroid. It is known that testosterone can be metabolized into dihydrotestosterone ("DHT") by an enzyme 5α-reductase and into estradiol ("E2") by an aromatase enzyme complex.

In a biological body, 98% of testosterone is circulating in the blood in a state of binding to a protein, and the remaining 2% of testosterone is circulating in the blood without binding to a protein, that is, as free testosterone.

In a male, about 40% of testosterone binding to a protein binds to the aforementioned high-affinity sex hormone-binding globulin (SHBG), and the remaining 60% of testosterone is present in a state of weakly binding to albumin.

Among testosterones, free testosterone and the testosterone weakly binding to albumin act as a physiologically effective sex hormone in the biological body. Therefore, the testosterone not binding to SHBG is called "physiologically effective testosterone".

Testosterone is available in the market, and examples thereof include testosterone from Wako Pure Chemical Industries, Ltd., and the like.

The content of testosterone in the composition for percutaneous absorption with respect to the total amount of the composition for percutaneous absorption is preferably from 0.5% by mass to 10% by mass.

In an example of an embodiment of the composition for percutaneous absorption, the content of testosterone is more preferably from 0.6% by mass to 8% by mass, even more preferably from 0.9% by mass to 7% by mass, and still more preferably from 0.9% by mass to 5% by mass.

The content of testosterone can be changed according to the use of the composition for percutaneous absorption. For example, in a case where the composition for percutaneous absorption is used as an embrocation, the content of testosterone with respect to the total amount of the composition is preferably within a range of from 0.5% by mass to 5% by mass. In a case where the composition for percutaneous absorption is used as a sticking agent, the content of testosterone with respect to the total amount of the composition is preferably within a range of from 0.5% by mass to 10% by mass. The embrocation and the sticking agent will be described later.

In a case where the composition for percutaneous absorption in which the content of testosterone is within the aforementioned range is applied to the skin, testosterone is expected to be able to show a sufficient efficacy, and the stability of the composition is excellent.

### [One or more organic solvents selected from group consisting of propylene glycol, 1,3-butylene glycol, dipropylene glycol, and polyethylene glycol]

In the composition of the present disclosure, the content of one or more organic solvents selected from the group consisting of PG, BG, DPG, and PEG with respect to the total amount of the composition for percutaneous absorption is 60% by mass to 90% by mass. The specific organic solvents are hydrophilic solvents which excellently dissolve testosterone and have affinity for water.

In a case where 0.5% by mass of testosterone is dissolved in the organic solvents at a temperature of 25°C and then left to stand for 24 hours, and then the precipitation of crystals is not observed, it can be said that the organic solvents excellently dissolves testosterone.

All of PG, BG, DPG, and PEG are known as organic solvents having excellent biocompatibility and hydrophobicity. These are organic solvents widely used in skin preparations for external use, cosmetics, and the like.

PEG is used as a solvent. Therefore, it is preferable to use PEG having an average molecular weight of equal to or less than 2,000. The average molecular weight of PEG is more preferably within a range of 180 to 1,600, and even more preferably within a range of 180 to 1,100.

Among the specific organic solvents, from the viewpoint of the solubility of testosterone, the composition for percutaneous absorption preferably contains one or more organic solvents selected from the group consisting of PG, BG, and DPG, more preferably contains one or more organic solvents selected from the group consisting of PG and BG, and even more preferably contains PG.

The composition may contain only one kind of organic solvent or two or more kinds of organic solvents selected from the group consisting of PG, BG, DPG, and PEG.

The total content of the specific organic solvents with respect to the total amount of the composition for percutaneous absorption is preferably equal to or greater than 60% by mass, more preferably equal to or greater than 65% by mass, and even more preferably equal to or greater than 70% by mass, because then the solubility of testosterone can be further improved. Furthermore, from the viewpoint of manufacturing properties in which a uniform composition can be easily manufactured, the total content of the specific organic solvents with respect to the total amount of the composition for percutaneous absorption is equal to or less than 90% by mass and preferably equal to or less than 85% by mass.

In the composition for percutaneous absorption, the total content of the specific organic solvents is 60% by mass to 90% by mass, preferably within a range of 65% by mass to 90% by mass, and even more preferably within a range of 70% by mass to 85% by mass.

In a case where the total content of the specific organic solvents is less than 60% by mass, sufficient solubility and skin permeability of testosterone are not easily obtained. In a case where the total content of the specific organic solvents is greater than 90% by mass, it is difficult to manufacture a uniform composition, and the manufacturing properties deteriorate in some cases.

The preferable content of each of the organic solvents in a case where PG, BG, DPG, and PEG are used as the specific organic solvents will be described. The content described below is a content of each of the specific organic solvents. The composition may contain one kind of specific organic solvent singly or two or more kinds of specific organic solvents, such that the total content of the specific organic solvents in the composition falls into the preferable range described above.

In a case where PG is used as a specific organic solvent, the content of PG with respect to the total amount of the composition is preferably within a range of 2% by mass to 90% by mass, and more preferably within a range of 5% by mass to 72% by mass.

In a case where BG is used as a specific organic solvent, the content of BG with respect to the total amount of the composition is preferably within a range of 2% by mass to 90% by mass, and more preferably within a range of 5% by mass to 45% by mass.

In a case where DPG is used as a specific organic solvent, the content of DPG with respect to the total amount of the composition is preferably within a range of 2% by mass to 90% by mass, more preferably within a range of 5% by mass to 25% by mass, and even more preferably within a range of 5% by mass to 10% by mass.

In a case where PEG is used as a specific organic solvent, the content of PEG with respect to the total amount of the composition is preferably within a range of 2% by mass to 90% by mass, more preferably within a range of 5% by mass to 70% by mass, and even more preferably within a range of 10% by mass to 45% by mass.

### [Water]

The composition for percutaneous absorption of the present disclosure can contain water.

In a case where the composition contains water as a solvent, as the water, for example, purified water, distilled water, deionized water, pure water, ultrapure water, water for injection, and the like are preferable, because these have excellent biocompatibility and contain a small amount of impurities.

In a case where the composition contains water as a solvent, the content of the water with respect to the total amount of the composition for percutaneous absorption is preferably within a range of 5% by mass to 35% by mass and more preferably within a range of 6% by mass to 33% by mass, because then the skin permeability of testosterone, the texture of the composition, and the like can be further improved.

In a case where the composition contains water as a solvent, provided that the content of the water is within the aforementioned range, the solubility of testosterone becomes better, and the manufacturing properties are further improved.

In some cases, for the purpose of adjusting pH and the like, 0.1 N (10³ mol/m³) sodium hydroxide or the like is used in the composition.

In a case where the composition contains water as a solvent, as the aforementioned content of the water, the amount of the water contained as a solvent plus the amount of water, for example, a 0.1 N aqueous sodium hydroxide solution and the like, used for adjusting other components contained in the composition, that is, the value of total content of water in the composition is adopted.

### [Monohydric alcohol having 2 to 4 carbon atoms]

The composition can contain a monohydric alcohol having 2 to 4 carbon atoms (hereinafter, referred to as a lower alcohol in some cases). Examples of the lower alcohol include ethyl alcohol, 1-propyl alcohol, 2-propyl alcohol, n-butyl alcohol, sec-butyl alcohol, tert-butyl alcohol, and the like. Among these, ethyl alcohol, 1-propyl alcohol, and the like are preferable.

In a case where the composition contains a lower alcohol, the solubility of testosterone in the composition can be further improved. In contrast, in a case where the composition containing a large amount of lower alcohol is applied to the skin, sometimes the composition causes skin irritation due to the alcohol. Furthermore, it is difficult to administer such a composition to a person sensitive to an alcohol.

Accordingly, in a case where a lower alcohol is used in the composition, the total content of the lower alcohol with respect to the total amount of the composition for percutaneous absorption is equal to or less than 10% by mass, preferably equal to or less than 4% by mass, more preferably equal to or less than 2% by mass, even more preferably equal to or less than 1% by mass, and most preferably equal to or less than 0.1% by mass. From the viewpoint of further reducing skin irritation, an aspect is also preferable in which the composition does not contain a monohydric alcohol having 2 to 4 carbon atoms.

### [Other additives]

Within a range that does not impair the effects of the present invention, the composition of the present disclosure may further contain various compounds (hereinafter, referred to as other additives in some cases) according to the purpose, in addition to testosterone, the specific organic solvents, and water which is an optional component.

Examples of other additives include a gelling agent, a skin absorption accelerator, a preservative, an antioxidant, a pH adjuster, a buffer, a colorant, a surfactant, a solubilizing agent, a pressure sensitive adhesive, an adhesion enhancer, a wetting agent, an emollient, a skin protective agent, a stabilizing agent, and the like.

### (Gelling agent)

The composition may contain a gelling agent. In a case where the composition contains a gelling agent, the stability can be further improved, and the physical properties suitable for the composition to be administered to the skin can be imparted.

Examples of the gelling agent that can be contained in the composition include polymer compounds known as thickeners of aqueous compositions and the like. As the gelling agent, the compound that can make an aqueous composition into a gel by adjusting the viscosity of the composition can be used without particular limitation.

Specifically, examples of the gelling agent include polymers such as cellulose derivatives (for example, hydroxypropyl cellulose, carboxymethyl cellulose, methyl cellulose, hydroxyethyl cellulose, ethyl cellulose, and hydroxypropyl methyl cellulose), acrylic acid-based polymers (for example, a carboxyvinyl polymer), polyvinyl alcohol, polyvinyl pyrrolidone, polyacrylic acid, a polyethylene-polypropylene copolymer, xanthone, dextran, pectin, and natural rubber. Furthermore, as the gelling agent, it is possible to use polymers containing a cross-linked structure in a molecule, such as cross-linked polyacrylic acid. Among these, as the gelling agent, at least any of hydroxypropyl cellulose and a carboxyvinyl polymer is preferable.

In a case where the composition contains the gelling agent, the composition may contain only one kind of gelling agent or two or more kinds of gelling agents. The content of the gelling agent with respect to the total amount of the composition can be appropriately adjusted according to the desired physical properties of the composition. Generally, the content of the gelling agent is preferably within a range of 0.1 % by mass to 20% by mass, more preferably within a range of 0.3% by mass to 3% by mass, and even more preferably within a range of 0.5% by mass to 1.2% by mass.

### (Skin absorption accelerator)

The composition for percutaneous absorption of the present disclosure may contain a skin absorption accelerator.

In a case where the composition contains the specific organic solvents in a specific amount, the solubility and skin permeability of testosterone are further improved. In a case where the composition further contains a skin absorption accelerator, the skin permeability of testosterone is further improved.

The skin absorption accelerator is not particularly limited as long as it can be used in pharmaceutical products-cosmetics and the like. Examples of the skin absorption accelerator include polyoxyethylene cetyl ether, polyoxyethylene lauryl ether, polyoxyethylene oleyl ether, polyoxyethylene stearyl ether, polyoxyethylene behenyl ether, polyoxyethylene cholesteryl ether, polyoxyethylene isocetyl ether, polyoxyethylene tridecyl ether, polyoxyethylene methyl glucoside, polyethylene glycol laurate, polyethylene glycol oleate, polyethylene glycol stearate, polyethylene glycol isostearate, polyoxyethylene polyoxypropylene glycol, polyoxyethylene hydrogenated castor oil, polyoxyethylene castor oil, polyoxyethylene sorbitan monolaurate, polyoxyethylene sorbitan monostearate, polyoxyethylene sorbitan tristearate, polyoxyethylene sorbitan monooleate, polyoxyethylene sorbitan monopalmitate, sorbitan monolaurate, sorbitan isostearate, sorbitan monooleate, sorbitan monopalmitate, sorbitan monostearate, sorbitan sesquioleate, sorbitan tristearate, glycerol monooleate, ethylhexyl salicylate, isopropyl myristate, diethyl sebacate, cetyl palmitate, diisopropyl adipate, diisobutyl adipate, diisopropyl dilinoleate, ethyl oleate, isostearyl isostearate, isopropyl palmitate, isopropyl stearate, methyl laurate, methyl stearate, oleyl oleate, myristyl lactate, oleic acid, sorbic acid, levulinic acid, isostearic acid, oleyl alcohol, cetyl alcohol, stearyl alcohol, isostearyl alcohol, myristyl alcohol, behenyl alcohol, sodium cocoyl sarcosinate, sodium laureth sulfate, dipotassium glycyrrhizinate, urea, dimethyl isosorbide, N-methyl pyrrolidone, isopropanol, aminomethyl propanol, benzyl alcohol, diethylene glycol monoethyl ether, dimethyl sulfoxide, decylmethyl sulfoxide, ethyl acetate, propylene carbonate, propylene glycol diacetate, cyclomethicone, gluconolactone, lemon oil, menthol, limonene, α-terpinenol, and the like. Among these, from the viewpoint of the biocompatibility and efficacy, polyoxyethylene oleyl ether, glycerol monooleate, methyl laurate, diethyl sebacate, ethylhexyl salicylate, menthol, isopropyl palmitate, oleic acid, oleyl alcohol, or isopropyl myristate is preferable, and isopropyl palmitate, isopropyl myristate, or oleyl alcohol is more preferable.

The composition may contain only one kind of skin absorption accelerator or two or more kinds of skin absorption accelerators.

In a case where the composition contains isopropyl myristate as an absorption accelerator, for example, provided that the content of isopropyl myristate with respect to the total amount of the composition for percutaneous absorption is equal to or less than 0.5% by mass which is a small amount, the effect of improving the skin permeability of testosterone can also be obtained.

In a case where the composition contains isopropyl myristate, the lower limit of the content of isopropyl myristate is not particularly limited. It is considered that in a case where the lower limit is equal to or greater than 0.1% by mass, the skin absorption accelerator contained in the composition can bring about effects.

The composition for percutaneous absorption of the present disclosure may contain a solubilizing agent. In a case where the composition contains a solubilizing agent, the solubility of testosterone is further improved.

Examples of the solubilizing agent include diethyl sebacate, diisopropyl adipate, α-terpineol, l-menthol, oleic acid, oleyl alcohol, and the like. From the viewpoint of the skin permeability and the efficacy of testosterone, diethyl sebacate, diisopropyl adipate, α-terpineol, or l-menthol is preferable.

The composition may contain only one kind of solubilizing agent or two or more kinds of solubilizing agents.

The composition may contain a preservative.

Examples of the preservative include benzalkonium chloride, benzoic acid, benzyl alcohol, bronopol, paraben, cetrimide, chlorhexidine, cresol, imidurea, phenol, phenoxyethanol, phenethyl alcohol, thimerosal, sorbic acid, salts and derivatives of these, and the like.

The composition may contain an antioxidant.

Examples of the antioxidant include tocopherol, ascorbic acid, butylated hydroxyanisole, butylated hydroxytoluene, fumaric acid, malic acid, citric acid, propyl gallate, sulfurous acid, edetic acid, salts and derivatives of these, and the like.

### <Preferable aspect of composition for percutaneous absorption>

### (Dosage form of composition for percutaneous absorption)

At the time of making the composition for percutaneous absorption of the present disclosure into a composition, the dosage form thereof is not particularly limited, and it is possible to adopt dosage forms such as an embrocation and a sticking agent.

Examples of the embrocation include a gel (an aqueous gel or an oil-based gel), a cream, an ointment, a liquid (a lotion or a liniment), and the like.

The sticking agent takes, for example, a form including a testosterone-containing pressure sensitive adhesive layer which is formed by coating the surface of an appropriate substrate with the composition further containing a pressure sensitive adhesive or an adhesive, and can adhere to the skin. Alternatively, the sticking agent takes a form in which the composition of the present disclosure containing testosterone is sealed into the space between a substrate and a release control layer that testosterone can permeate.

The constitution of the sticking agent having the aforementioned release control layer is described, for example, in paragraphs "0007" to "0022" and Fig. 1 in JP2003-063954A, and can be referred to in the present disclosure.

### (Preferable viscosity of composition for percutaneous absorption)

The viscosity of the composition for percutaneous absorption measured at a shear rate of 100 s⁻¹ and 25°C is preferably within a range of 0.5 Pa·s to 5 Pa·s, and more preferably within a range of 1 Pa·s to 4 Pa·s.

The viscosity can be measured under the standard conditions based on the method described in the 16^{th} revised edition of Japanese pharmacopeia by using a cone plate-type viscometer.

In a case where the dosage form of the composition is a gel or a cream, provided that the viscosity is within the above range, when the composition is administered to the skin, the composition exhibits higher affinity for the skin, spreads better on the skin, and exhibits better adhesiveness by which the composition stably remains on the skin.

The viscosity of the composition can be adjusted by known methods. Examples of the viscosity adjusting methods include a method of adjusting at least any of the type and content of the aforementioned organic solvents, a method of adjusting at least any of the type and content of the gelling agent (viscosity adjuster), a method of adjusting a salt concentration, and the like.

In a case where the composition is used as a lotion or an ointment, sometimes a preferable viscosity of the composition is not within the aforementioned preferable range of viscosity.

### (pH of composition for percutaneous absorption)

The pH of the composition for percutaneous absorption at 25°C, which is expressed by a value obtained by measuring the pH of a diluted solution prepared by diluting 0.1 g of the composition with 1 ml of water, is preferably within a range of 3.0 to 8.0, and more preferably within a range of 3.5 to 6.0.

In a case where the pH of the composition is within the above range, the stability of the composition is further improved, and the skin irritation is further reduced.

The pH of the composition can be controlled by known methods. Examples of the pH control method for the composition include a method of using a pH adjuster, a method of using a buffer, and the like.

### (Skin permeability of testosterone)

The composition for percutaneous absorption of the present disclosure has the aforementioned constitution. Therefore, the composition has excellent skin permeability of testosterone. The skin permeability can be evaluated by an in-vitro skin permeation experiment method, an in-vivo skin permeation experiment method, and the like.

Examples of the in-vitro skin permeation experiment method include a method of using a diffusion cell. Examples of the diffusion cell include a vertical cell such as a Franz diffusion cell, a horizontal cell, and the like. The diffusion cell includes two cell parts, and is used with a membrane for measuring permeability interposed between the two cell parts. Examples of the membrane include the human skin, the animal skin, a 3D culture skin model, an artificial membrane, and the like. The skin permeability of testosterone contained in the composition can be evaluated, for example, by a skin permeability test using the skin isolated from a hairless rat shown in the example of an evaluation method described below.

### (Example of evaluation method)

The skin isolated from an 8-week-old specific pathogen free (SPF) hairless rat (Ishikawa Laboratory Animals) is installed in a Franz diffusion cell (PermeGear, Inc, a jacketed stationary type, effective permeation area: 1 cm², receptor volume: 8 mL) such that the horny layer of the skin faces up. The horny layer side on top of the skin was uniformly coated with the composition as an evaluation subject at 4 mg/cm², and the level of testosterone eluted through the skin into the receptor fluid with which the cell is filled is measured. In the diffusion cell, water at a temperature of 32°C is caused to circulate in the jacket such that the surface temperature of the skin is maintained. As the receptor fluid, a phosphate buffer solution with pH 7.4 is used. The receptor fluid is stirred with a magnetic stirrer in an unclosed state. After 24 hours, the receptor fluid in the diffusion cell is collected and replaced with a new receptor fluid having the same volume as that of the collected one.

The collected receptor fluid is analyzed by high-performance liquid chromatography (Alliance HPLC system: trade name, WATERS), and the level of testosterone in the receptor fluid is measured.

In a case where the amount of testosterone permeating the skin for 24 hours is equal to or greater than 4 µg/cm², the composition is evaluated as a preparation (composition) having sufficient skin permeability.

Examples of the in-vivo skin permeation experiment method include a pharmacokinetic test for skin, a biological test, a residual amount test, a kinetic test, a clinical test, an aminal test, an exposure amount test, and the like.

### <Use of composition for percutaneous absorption>

The composition for percutaneous absorption is used by being administered to the skin of a subject of application. In a case where the composition is in the form of a lotion, an aqueous gel, an ointment, or a cream, the composition is administered by being applied to or placed on the skin of the subject of application. In some cases, the composition administered onto the skin dries over time.

The region of the skin to which the composition is administered is not particularly limited. However, from the viewpoint of making sure that testosterone demonstrates the efficacy, it is preferable to administer the composition to the outside of both thighs, the shoulders, the abdomen, or the hip of the subject of application. The composition may be administered using fingers or a spatula. Alternatively, the composition may be directly administered to the skin from a container including a dispenser. In a case where the composition is administered using fingers, it is preferable to wash the fingers well after the administration such that testosterone is inhibited from being absorbed into an undesired region of the skin.

The composition of the present disclosure has excellent skin permeability of testosterone. Accordingly, by the administration of the composition, testosterone rapidly permeates the skin, and the level of testosterone in the blood increases to be within a desired range. Therefore, the symptoms resulting from hypotestosteronism is expected to be able to be relieved or recovered.

The single dose of the composition and the number of doses per day of the composition can be appropriately selected according to the purpose. That is, in order for a necessary amount of testosterone to be able to be absorbed into the skin of the subject of application, the dose and the number of doses are selected. Generally, the daily dose is about 1 g to 10 g, and the number of doses per day is about 1 to 3. However, the dose and the number of doses are not limited to the above range.

The form of packing the composition is not particularly limited. The composition may be individually packaged by the daily dose. Alternatively, any of a bottle with or without a dispenser filled with the composition in such an amount that enables the composition to be administered multiple times, a tube containing the composition in such an amount that enables the composition to be administered multiple times, and the like may be adopted.

The packing container is not particularly limited as long as the composition to be contained in the interior of the container, particularly, testosterone can be stably retained. Examples of the packing container include a container or a packing bag formed of a resin such as polyethylene or polypropylene, a container or a packing bag formed of a resin laminated with a metal such as aluminum, and the like. Furthermore, a glass container and the like can also be used.

The sticking agent such as a tape, a poultice, or a patch can be obtained, for example, by forming a layer containing the composition on one surface of an appropriate substrate or by sandwiching the composition between an appropriate substrate and a release control layer provided on the substrate. Examples of the substrate which can be used in the sticking agent include a resin film, non-woven cloth, or cloth having air permeability, a film not having air permeability, and the like. For preparing the sticking agent, a substrate selected from the above according to the purpose of use of the composition can be used.

The sticking agent can be prepared by disposing the composition on one surface of a substrate. The side on which the composition is disposed becomes a surface adhering to the skin. The surface adhering to the skin may include a pressure sensitive adhesive layer or an adhesive layer that have biocompatibility. The pressure sensitive adhesive layer or the adhesive layer may be a layer containing the composition as described above, or may be a layer provided separately from the layer containing the composition. The sticking agent can be fixed by being stuck to the skin by using an adhesive or a pressure sensitive adhesive. In a case where the sticking agent does not include an adhesive or a pressure sensitive adhesive, the sticking agent can be used by fixing a medical patch to the skin by using a tape or the like.

In a case where the composition is an aqueous gel or an ointment, by directly applying the composition to a substrate, a sticking agent can be formed. In a case where the composition is a low-viscosity composition such as a lotion, by impregnating a support such as non-woven cloth with the composition and fixing the support impregnated with the composition onto the surface of a substrate, a sticking agent can be formed. In the sticking agent, the region where the support impregnated with the composition is present may be covered with the aforementioned release control layer.

In a case where the composition is used in the form of a sticking agent, from the region to which the sticking agent fixed, testosterone continuously permeates the skin and supplied into the body for a certain period of time.

### [Composition for percutaneous absorption mixed with high level of testosterone]

Unlike the embrocation, the sticking agent can supply a drug into the body for a long period of time. However, the area of the sticking agent that can be applied to the skin is limited to some extent. Therefore, as the composition used in the sticking agent, it is preferable to prepare a composition for percutaneous absorption mixed with testosterone of a level higher than the level of testosterone mixed with the embrocation. As described above, the content of testosterone in the composition with respect to the total amount of the composition is preferably from 0.5% by mass to 10% by mass. However, in a case where the composition is used in a sticking agent, for example, by raising the level of testosterone in the composition such that the testosterone level is within a range of 2% by mass to 10% by mass, it is possible to prepare a sticking agent retaining a sufficient amount of drug in a limited area.

### [Method for manufacturing composition for percutaneous absorption]

The method for manufacturing the composition for percutaneous absorption of the present disclosure is not particularly limited, and the composition can be manufactured according to known methods.

As one of the manufacturing methods, for example, there is a manufacturing method including preparing a testosterone solution by stirring and mixing together testosterone, the specific organic solvents, and other additives soluble in an organic solvent that are added as desired, preparing an aqueous gel by stirring and mixing together water and an aqueous component such as a gelling agent added as desired, and preparing an aqueous gel-like composition containing testosterone by uniformly mixing the obtained testosterone solution with the obtained aqueous gel.

The composition for percutaneous absorption has excellent solubility and skin permeability of testosterone, and is inhibited from causing skin irritation. Therefore, the composition is useful for relieving or treating various symptoms resulting from a lack of testosterone.

The composition for percutaneous absorption of the present disclosure has excellent solubility and skin permeability of testosterone, and is inhibited from causing skin irritation. Therefore, a method in which the composition for percutaneous absorption is used is provided as another embodiment of the present invention.

For example, by administering the composition for percutaneous absorption described in the present specification to a patient in need of the composition for percutaneous absorption, a method for treating diseases resulting from hypotestosteronism is provided.

For example, there is provided a method for treating sexual dysfunction by administering the composition for percutaneous absorption as testosterone into the body at an effective dose. The method is expected to bring about effect of inhibiting various physiological changes resulting from sexual dysfunction, for example, a decrease in sex drive, impotence, a reduction in muscle mass, a reduction in bone density, depression, a decrease in strength, and a cognitive decline.

In a case where testosterone is percutaneously administered by using the composition for percutaneous absorption of the present disclosure, there are no fears of the low absorptivity in the gastrointestinal tract, the reduction in the effective dose caused by the metabolism in the liver, and the like that result from the oral administration, and the level of testosterone in the blood can be controlled within a desired range.

### Examples

Hereinafter, the present invention will be specifically described based on examples, but the present invention is not limited to following examples. In the following examples, unless otherwise specified, "%" means "% by mass".

### [Examples 1 to 24 and Comparative Examples 1 to 7]

According to the makeup shown in Tables 1 and 2, first, by stirring testosterone, organic solvents, and isopropyl myristate together in a container, a testosterone solution was prepared. The numerical values described in Tables 1 and 2 represent the content (% by mass) of each component with respect to the total amount of the composition. Furthermore, the blank in each of the following tables shows that the composition does not contain the corresponding component.

In another container, a carboxyvinyl polymer as a gelling agent in the amount described in Tables 1 and 2 was dissolved in water for injection in the amount described in Tables 1 and 2, thereby preparing an aqueous gel.

By using a rotation and revolution mixer (AWATORI RENTARO ARE-310, THINKY), the testosterone solution and the aqueous gel obtained as above were mixed together until the mixture appeared to be uniform, thereby obtaining an aqueous gel-like composition for percutaneous absorption. The rotation and revolution mixer used in the examples is a type of general stirring device which does not directly shear the content with a stirring blade.

The details of each component described in Tables 1 and 2 are as below.
- Testosterone (Wako Pure Chemical Industries, Ltd.)
- Propylene glycol (Wako Pure Chemical Industries, Ltd.)
- 1,3-Butylene glycol (manufactured by Daicel Corporation)
- Dipropylene glycol (Wako Pure Chemical Industries, Ltd.)
- Polyethylene glycol (trade name: MACROGOL 400 (NOF CORPORATION, molecular weight: 400)
- Polyethylene glycol (trade name: POLYETHYLENE GLYCOL 1000, Wako Pure Chemical Industries, Ltd., molecular weight: 1,000)
- Ethanol (Wako Pure Chemical Industries, Ltd.)
- Water (water for injection)
- Isopropyl myristate (Wako Pure Chemical Industries, Ltd.)
- l-menthol (Alfa Aesar)
- α-Terpineol (Wako Pure Chemical Industries, Ltd.)
- Carboxyvinyl polymer (CARBOPOL (registered trademark) 980NF, The Lubrizol Corporation)

### [Evaluation]

The compositions for percutaneous absorption of Examples 1 to 24 and Comparative Examples 1 to 7 obtained as above were evaluated as below.

### (1) Solubility of testosterone

By visually observing the composition for percutaneous absorption obtained immediately after the mixing of the testosterone solution with the aqueous gel and the composition for percutaneous absorption left as it was for 1 day at 25°C after the mixing, whether or not the crystal precipitation occurred was checked.
A: The crystal precipitation was not visually observed immediately after the mixing and after 1 day, and the composition was uniform.
B: The crystal precipitation was visually observed immediately after the mixing or after 1 day.

The results are shown in Tables 1 and 2.

**[Table 1]**

| | Formulation of composition for percutaneous absorption (% by mass) | | | | | | | | | | | | Evaluation result |
|---|---|---|---|---|---|---|---|---|---|---|---|---|---|
| | Testosterone | Ethanol | Propylene glycol | Dipropylene glycol | 1,3-Butylene glycol | Polyethylene glycol 400 | Glycerin | Isopropyl myristate | Carboxyvinyl polymer | 0.1 N sodium hydroxide | Water | Total | Uniformity |
| Comparative Example 1 | 1 | 67 | | | | | | 0.5 | 0.9 | 4.72 | 25.88 | 100 | A |
| Comparative Example 2 | 1 | 67 | | | | | | | 0.9 | 4.72 | 26.38 | 100 | A |
| Example 1 | 0.5 | | 67 | | | | | | 0.9 | 4.72 | 26.88 | 100 | A |
| Example 2 | 1 | | | 67 | | | | | 0.9 | 4.72 | 26.38 | 100 | A |
| Example 3 | 1 | | | | 67 | | | | 0.9 | 4.72 | 26.38 | 100 | A |
| Example 4 | 1 | | 75 | | | | | | 0.9 | 4.72 | 18.38 | 100 | A |
| Example 5 | 1 | | | 75 | | | | | 0.9 | 4.72 | 18.38 | 100 | A |
| Example 6 | 1.62 | | | 75 | | | | | 0.9 | 4.72 | 17.76 | 100 | A |
| Example 7 | 1 | | | | 75 | | | | 0.9 | 4.72 | 18.38 | 100 | A |
| Example 8 | 1.62 | | | | 75 | | | | 0.9 | 4.72 | 17.76 | 100 | A |
| Comparative Example 3 | 0.5 | | | | | | 75 | | 0.9 | 4.72 | 18.88 | 100 | B |
| Comparative Example 4 | 0.5 | | 50 | | | | | | 0.9 | 4.72 | 43.88 | 100 | B |
| Comparative Example 5 | 0.5 | | | 50 | | | | | 0.9 | 4.72 | 43.88 | 100 | B |
| Comparative Example 6 | 0.5 | | | | 50 | | | | 0.9 | 4.72 | 43.88 | 100 | B |
| Comparative Example 7 | 0.5 | | | | | 50 | | | 0.9 | 4.72 | 43.88 | 100 | B |

**[Table 2]**

| | Formulation of composition for percutaneous absorption (% by mass) | | | | | | | | | | | | | | | | Evaluation result |
|---|---|---|---|---|---|---|---|---|---|---|---|---|---|---|---|---|---|
| | Testosterone | Ethanol | Propylene glycol | Dipropylene glycol | 1,3-Butylene glycol | Polyethylene glycol 400 | Polyethylene glycol 1000 | Glycerin | Isopropyl myristate | Polysorbate 80 | α-Terpineol | l-Menthol | Carboxyvinyl polymer | 0.1 N sodium hydroxide | Water | Total | Uniformity |
| Example 9 | 1 | | 5 | 20 | 45 | | | | | | | | 0.9 | 4.72 | 23.38 | 100 | A |
| Example 10 | 1 | | 10 | 20 | 45 | | | | | | | | 0.9 | 4.72 | 18.38 | 100 | A |
| Example 11 | 1 | | 10 | 20 | 45 | | | | | | | | 0.7 | 3.67 | 19.63 | 100 | A |
| Example 12 | 1.62 | | 10 | 20 | 45 | | | | | | | | 0.9 | 4.72 | 17.76 | 100 | A |
| Example 13 | 1 | | 15 | 20 | 45 | | | | | | | | 0.9 | 4.72 | 13.38 | 100 | A |
| Example 14 | 1.62 | | 15 | 20 | 45 | | | | | | | | 0.9 | 4.72 | 12.76 | 100 | A |
| Example 15 | 1 | | 45 | | | 30 | | | | | | | 0.9 | 4.72 | 18.38 | 100 | A |
| Example 16 | 1 | | | 30 | | 45 | | | | | | | 0.9 | 4.72 | 18.38 | 100 | A |
| Example 17 | 1 | | | | 30 | 45 | | | | | | | 0.9 | 4.72 | 18.38 | 100 | A |
| Example 18 | 1 | | 45 | | | | 30 | | | | | | 0.9 | 4.72 | 18.38 | 100 | A |
| Example 19 | 1.62 | | 50 | | | | 30 | | 0.5 | 2 | | | 0.9 | 4.72 | 10.26 | 100 | A |
| Example 20 | 1.62 | | 50 | 10 | | | 20 | | 0.5 | 2 | | | 0.9 | 4.72 | 10.26 | 100 | A |
| Example 21 | 1.62 | | 50 | | 10 | | 20 | | 0.5 | 2 | | | 0.9 | 4.72 | 10.26 | 100 | A |
| Example 22 | 1.62 | | 50 | | | | 25 | | | | 5 | | 0.9 | 4.72 | 12.76 | 100 | A |
| Example 23 | 1.62 | | 50 | | | | 25 | | | | | 5 | 0.9 | 4.72 | 12.76 | 100 | A |
| Example 24 | 1.62 | | 40 | | | | 30 | 2 | 0.5 | | 5 | | 0.9 | 4.72 | 15.26 | 100 | A |

From the results shown in Tables 1 and 2, it was understood that all of the compositions for percutaneous absorption of the examples have excellent solubility of testosterone and are obtained as uniform compositions, and even though ethanol known to have a problem of skin irritation is not used, the solubility of testosterone in these compositions is the same as that in Comparative Examples 1 and 2 in which ethanol is used as a solvent.

On the contrary, in the composition for percutaneous absorption of Comparative Example 3 in which glycerin was used as a comparative organic solvent and the compositions for percutaneous absorption of Comparative Examples 4 to 7 in which the specific organic solvents were used but the content thereof was outside the range of the present invention, the precipitation of testosterone was observed, and these compositions were not obtained as uniform compositions.

### [Examples 25 to 27]

Example 25 was prepared in the same manner as in Example 10, except that in Example 10, 2% by mass of polysorbate 80 and 0.5% by mass of isopropyl myristate were further added, and the content of the water for injection was changed to 15.88% by mass.

A composition for percutaneous absorption of Example 26 was prepared in the same manner as in Example 15, except that in Example 15, 2% by mass of polysorbate 80 and 0.5% by mass of isopropyl myristate were further added, and the content of the water for injection was changed to 15.88% by mass.

A composition for percutaneous absorption of Example 27 was prepared in the same manner as in Example 18, except that in Example 18, 2% by mass of polysorbate 80 and 0.5% by mass of isopropyl myristate were further added, and the content of the water for injection was changed to 15.88% by mass.

The unit of the content of each component in the compositions described in Table 3 is % by mass.

Next, the compositions for percutaneous absorption of Example 25 to 27 obtained as above and the compositions for percutaneous absorption of Examples 4, 7, 10 and Comparative Examples 1 and 2 were evaluated regarding the skin permeability of testosterone by the method described below. The results are shown in Table 3.

### (2) Evaluation of skin permeability of testosterone

The skin isolated from an 8-week-old SPF hairless rat (Ishikawa Laboratory Animals) was installed in a jacketed Franz diffusion cell (PermeGear, Inc, a jacketed stationary type, effective permeation area: 1 cm², receptor volume: 8 mL) filled with a receptor fluid such that the horny layer of the skin faced up. In other words, the skin was installed such that the horny layer became a side that did not come into contact with the receptor fluid with which the diffusion cell was filled. The horny layer side on top of the skin was uniformly coated with the composition as an evaluation subject at 4 mg/cm², and the level of testosterone eluted through the skin into the receptor fluid with which the cell was filled was measured.

In the diffusion cell, water at a temperature of 32°C was caused to circulate in the jacket such that the surface temperature of the skin installed in the diffusion cell was maintained. As the receptor fluid, a phosphate buffer solution with pH 7.4 was used. The receptor fluid was stirred with a magnetic stirrer in an unclosed state. After 24 hours, 0.3 mL of the receptor fluid was collected.

The level of testosterone in the collected receptor fluid was measured by high-performance liquid chromatography (Alliance HPLC system: trade name, WATERS). The results are shown in Table 3.

**[Table 3]**

| | | Comparative Example 1 | Comparative Example 2 | Example 4 | Example 7 | Example 10 | Example 25 | Example 26 | Example 27 |
|---|---|---|---|---|---|---|---|---|---|
| Formulation of composition for percutaneous absorption (% by mass) | Testosterone | 1 | 1 | 1 | 1 | 1 | 1 | 1 | 1 |
| | Ethanol | 67 | 67 | | | | | | |
| | Propylene glycol | | | 75 | | 10 | 10 | 45 | 45 |
| | Dipropylene glycol | | | | | 20 | 20 | | |
| | 1,3-Butylene glycol | | | | 75 | 45 | 45 | | |
| | Polyethylene glycol 400 | | | | | | | 30 | |
| | Polyethylene glycol, 1000 | | | | | | | | 30 |
| | Isopropyl myristate | 0.5 | | | | | 0.5 | 0.5 | 0.5 |
| | Polysorbate 80 | | | | | | 2 | 2 | 2 |
| | Carboxyvinyl polymer | 0.9 | 0.9 | 0.9 | 0.9 | 0.9 | 0.9 | 0.9 | 0.9 |
| | 0.1 N sodium hydroxide | 4.72 | 4.72 | 4.72 | 4.72 | 4.72 | 4.72 | 4.72 | 4.72 |
| | Water | 25.88 | 26.38 | 18.38 | 18.38 | 18.38 | 15.88 | 15.88 | 15.88 |
| | Total | 100 | 100 | 100 | 100 | 100 | 100 | 100 | 100 |
| Evaluation result | Amount of permeating skin (µg/cm², 24 hr) | 5.65 | 5.09 | 15.29 | 8.81 | 5.60 | 9.56 | 15.48 | 11.74 |

From Table 3, it was understood that the skin permeability of testosterone is higher in Examples 4, 7, 10, 25, 26, and 27, in which the specific organic solvents are used, than in Comparative Examples 1 and 2 in which ethanol is used as a solvent, and the skin permeability is particularly higher in Example 4 containing PG than in other examples.

The skin permeability of the composition for percutaneous absorption of Example 10, in which isopropyl myristate as a skin absorption accelerator was not used, was found to be the same as the skin permeability of the composition for percutaneous absorption of Comparative Example 1 in which isopropyl myristate was added to ethanol. Furthermore, as is evident from the comparison between Example 10 and Example 25, in a case where the composition contains isopropyl myristate as a skin absorption accelerator, the skin permeability is further improved.

### [Examples 28 and 29 and Comparative Examples 8 to 10]

By changing the content of propylene glycol as a specific organic solvent in Example 4 to the amount described in Table 4, compositions for percutaneous absorption of Examples 28 and 29 and Comparative Examples 8 to 10 were prepared. The unit of the content of each component in the compositions described in Table 4 is % by mass.

Next, the obtained compositions for percutaneous absorption of Examples 28, 29, and 4 and Comparative Examples 8 to 10 were evaluated regarding the manufacturing properties of the testosterone-containing composition for percutaneous absorption by the method described below.

The results are shown in Table 4.

### (3) Evaluation of manufacturing properties of testosterone-containing gel composition

By visually observing the composition for percutaneous absorption obtained immediately after mixing the testosterone solution with the aqueous gel or a carboxyvinyl polymer by using a rotation and revolution mixer, whether the aqueous gel was uniformly dispersed was evaluated.
A: The aqueous gel was uniformly dispersed, and the composition was uniform.
B: The aqueous gel or the carboxyvinyl polymer was not dispersed, and the composition was not uniform.

**[Table 4]**

| | | Example 4 | Example 28 | Example 29 | Comparative Example 8 | Comparative Example 9 | Comparative Example 10 |
|---|---|---|---|---|---|---|---|
| Formulation of composition for percutaneous absorption (% by mass) | Testosterone | 1 | 1 | 1 | 1 | 1 | 1 |
| | Propylene glycol | 75 | 80 | 85 | 93.38 | 98.1 | 96.5 |
| | Carboxyvinyl polymer | 0.9 | 0.9 | 0.9 | 0.9 | 0.9 | 2.5 |
| | 0.1 N sodium hydroxide | 4.72 | 4.72 | 4.72 | 4.72 | | |
| | Water | 18.38 | 13.38 | 8.38 | | | |
| | Total | 100 | 100 | 100 | 100 | 100 | 100 |
| Evaluation result | Manufacturing properties | A | A | A | B | B | B |

From the results shown in Table 4, it is understood that the manufacturing properties of the compositions of the examples are excellent. In contrast, it is understood that by means of mixing using a generally used rotation and revolution mixer, the compositions for percutaneous absorption of Comparative Examples 8 to 10, in which the content of the specific organic solvents is greater than 90% by mass with respect to the total amount of each composition, are not obtained as uniform gel-like compositions for percutaneous absorption, and the manufacturing properties of these compositions are poor compared to the examples.

### [Examples 30 to 39 and Comparative Example 11]

According to the makeup shown in Table 5, compositions for percutaneous absorption of Examples 30 to 39 and Comparative Example 11 were manufactured by the same method as the method used in Example 1, and these compositions were evaluated regarding the solubility of testosterone in the same manner as in Examples 1 to 24.

**[Table 5]**

| | | Comparative Example 11 | Example 30 | Example 31 | Example 32 | Example 33 | Example 34 | Example 35 | Example 36 | Example 37 | Example 38 | Example 39 |
|---|---|---|---|---|---|---|---|---|---|---|---|---|
| Formulation of composition for percutaneous absorption (% by mass) | Testosterone | 5 | 6 | 5 | 4 | 3 | 5 | 5 | 5 | 5 | 5 | 5 |
| | Ethanol | 67 | | | | | | | | | | |
| | Propylene glycol | | 20 | 20 | 21 | 22 | 35 | 70 | 70 | 20 | | |
| | Dipropylene glycol | | 13.5 | 14.5 | 14.5 | 14.5 | | 5 | | 10.5 | | |
| | 1,3-Butylene glycol | | 45 | 45 | 45 | 45 | 44.5 | | | 45 | 80 | 80 |
| | Isopropyl myristate | 0.5 | | | | | | | | | | |
| | Isopropyl palmitate | | 0.5 | 0.5 | 0.5 | 0.5 | 0.5 | 0.5 | | 0.5 | | |
| | Diethyl sebacate | | 5 | 5 | 5 | 5 | 3 | 1 | | 5 | 5 | 5 |
| | Diisopropyl adipate | | | | | | 2 | | | | | |
| | α-Terpineol | | | | | | | 10 | 15 | | | |
| | l-Menthol | | | | | | | | | 4 | | |
| | Hydroxypropyl cellulose | | | | | | | | | | | 2 |
| | Carboxyvinyl polymer | 0.9 | 0.9 | 0.9 | 0.9 | 0.9 | 0.9 | 0.9 | 0.9 | 0.9 | 0.9 | |
| | 0.1 N sodium hydroxide | 4.72 | 4.72 | 4.72 | 4.72 | 4.72 | 4.72 | 4.72 | 4.72 | 4.72 | 4.72 | |
| | Water | 21.88 | 4.38 | 4.38 | 4.38 | 4.38 | 4.38 | 2.88 | 4.38 | 4.38 | 4.38 | 8 |
| | Total | 100 | 100 | 100 | 100 | 100 | 100 | 100 | 100 | 100 | 100 | 100 |
| Evaluation result | Uniformity | A | A | A | A | A | A | A | A | A | A | A |

From the results shown in Table 5, it is understood that a high level of testosterone can be mixed with all of the compositions for percutaneous absorption of the examples, these compositions are obtained as uniform compositions, and even though ethanol known to have a problem of skin irritation is not used, the solubility of testosterone in these compositions is the same as that in the comparative example in which ethanol is used as a solvent.

### [Examples 40 to 52 and Comparative Example 11]

The skin permeability of testosterone in the compositions for percutaneous absorption of Examples 35 to 37 and 40 to 52 and Comparative Example 11 was evaluated by the same method as the method used in Example 25, except that compositions for percutaneous absorption of Examples 40 to 52 were manufactured by the same method as the method used in Example 1 according to the makeup described in Tables 6 to 8 shown below, and the skin was uniformly coated with the composition as an evaluation subject at 300 mg/cm².

**[Table 6]**

| | | Comparative Example 11 | Example 36 | Example 40 | Example 41 | Example 42 | Example 43 | |
|---|---|---|---|---|---|---|---|---|
| | Testosterone | 5 | 5 | 5 | 5 | 5 | 5 | |
| | Ethanol | 67 | | | | | | |
| | Propylene glycol | | 70 | 70 | 70 | 70 | 70 | |
| | Dipropylene glycol | | | | | | | |
| | 1,3-Butylene glycol | | | | | | | |
| | α-Terpineol | | 15 | 15 | 15 | 15 | 15 | |
| | Polyoxyethylene (2) oleyl ether | | | 0.5 | | | | |
| Formulation | Polyoxyethylene (7) oleyl ether | | | | 0.5 | | | |
| of | Glycerol monooleate | | | | | 0.5 | | |
| composition for | Methyl laurate | | | | | | 0.5 | |
| percutaneous | Diethyl sebacate | | | | | | | |
| absorption | Isopropyl myristate | 0.5 | | | | | | |
| (% by mass) | Ethyl hexyl salicylate | | | | | | | |
| | Oleic acid | | | | | | | |
| | Oleyl alcohol | | | | | | | |
| | Isopropyl palmitate | | | | | | | |
| | l-Menthol | | | | | | | |
| | Carboxyvinyl polymer | 0.9 | 0.9 | 0.9 | 0.9 | 0.9 | 0.9 | |
| | 0.1 N sodium hydroxide | 4.72 | 4.72 | 4.72 | 4.72 | 4.72 | 4.72 | |
| | Water | 21.88 | 4.38 | 3.88 | 3.88 | 3.88 | 3.88 | |
| | Total | 100 | 100 | 100 | 100 | 100 | 100 | |
| Evaluation result | Amount of permeating skin (µg/cm², 24 hr) | 361.2 | 398.4 | 479.2 | 446.5 | 541.4 | 631.8 | |

**[Table 7]**

| | | Example 44 | Example 45 | Example 46 | Example 47 | Example 48 | Example 49 |
|---|---|---|---|---|---|---|---|
| | Testosterone | 5 | 5 | 5 | 5 | 5 | 5 |
| | Ethanol | | | | | | |
| | Propylene glycol | 70 | 70 | 70 | 70 | 70 | 70 |
| | Dipropylene glycol | | | | | | |
| | 1,3-Butylene glycol | | | | | | |
| | α-Terpineol | 15 | 15 | 15 | 15 | 15 | 15 |
| | Polyoxyethylene (2) oleyl ether | | | | | | |
| Formulation | Polyoxyethylene (7) oleyl ether | | | | | | |
| of composition | Glycerol monooleate | | | | | | |
| for | Methyl laurate | | | | | | |
| percutaneous | Diethyl sebacate | 0.5 | | | | | |
| absorption | Isopropyl myristate | | 0.5 | | | | |
| (% by mass) | Ethyl hexyl salicylate | | | 0.5 | | | |
| | Oleic acid | | | | 0.5 | | |
| | Oleyl alcohol | | | | | 0.5 | |
| | Isopropyl palmitate | | | | | | 0.5 |
| | l-Menthol | | | | | | |
| | Carboxyvinyl polymer | 0.9 | 0.9 | 0.9 | 0.9 | 0.9 | 0.9 |
| | 0.1 N sodium hydroxide | 4.72 | 4.72 | 4.72 | 4.72 | 4.72 | 4.72 |
| | Water | 3.88 | 3.88 | 3.88 | 3.88 | 3.88 | 3.88 |
| | Total | 100 | 100 | 100 | 100 | 100 | 100 |
| Evaluation result | Amount of permeating skin (µg/cm², 24 hr) | 605.5 | 597.0 | 666.8 | 667.2 | 726.5 | 888.2 |

**[Table 8]**

| | | Comparative Example 11 | Example 36 | Example 50 | Example 51 | Example 52 | Example 35 | Example 37 |
|---|---|---|---|---|---|---|---|---|
| | Testosterone | 5 | 5 | 5 | 5 | 5 | 5 | 5 |
| | Ethanol | 67 | | | | | | |
| | Propylene glycol | | 70 | 70 | 70 | 70 | 70 | 20 |
| | Dipropylene glycol | | | 5 | 5 | 5 | 5 | 10.5 |
| | 1,3-Butylene glycol | | | | | | | 45 |
| | α-Terpineol | | 15 | 10 | 10 | 10 | 10 | |
| | Polyoxyethylene (2) oleyl ether | | | | | | | |
| | Polyoxyethylene (7) oleyl ether | | | | | | | |
| Formulation | Glycerol monooleate | | | | | | | |
| of composition | Methyl laurate | | | | | | | |
| for | Diethyl sebacate | | | 1 | 1 | 1 | 1 | 5 |
| percutaneous absorption | Isopropyl myristate | 0.5 | | | | | | |
| (% by mass) | Ethyl hexyl salicylate | | | | | | | |
| | Oleic acid | | | | 0.5 | | | |
| | Oleyl alcohol | | | | | 0.5 | | |
| | Isopropyl palmitate | | | | | | 0.5 | 0.5 |
| | l-Menthol | | | | | | | 4 |
| | Carboxyvinyl polymer | 0.9 | 0.9 | 0.9 | 0.9 | 0.9 | 0.9 | 0.9 |
| | 0.1 N sodium hydroxide | 4.72 | 4.72 | 4.72 | 4.72 | 4.72 | 4.72 | 4.72 |
| | Water | 21.88 | 4.38 | 3.38 | 2.88 | 2.88 | 2.88 | 4.38 |
| | Total | 100 | 100 | 100 | 100 | 100 | 100 | 100 |
| Evaluation result | Amount of permeating skin (µg/cm², 24 hr) | 361.2 | 398.4 | 524.5 | 632.4 | 781.5 | 1049.1 | 385.6 |

From Tables 6 to 8, it is understood that in the compositions for percutaneous absorption containing the specific organic solvents mixed with a high level of testosterone, the skin permeability of testosterone is higher than in Comparative Example 11 in which ethanol is used as a solvent. Furthermore, it is understood that the addition of various skin absorption accelerators further improves the skin permeability.

The disclosures of JP2015-194354 filed on September 30, 2015 and JP2016-139690 filed on July 14, 2016 are incorporated into the present specification by reference.

All of the documents, the patent applications, and the technical standards described in the present specification are incorporated into the present specification by reference, as if each of the documents, the patent applications, and the technical standards is specifically and individually is described by reference.

## Claims

1. A composition for percutaneous absorption, comprising:
testosterone; and
one or more organic solvents selected from the group consisting of propylene glycol, 1,3-butylene glycol, dipropylene glycol and polyethylene glycol,
wherein a total content of the organic solvents with respect to a total amount of the composition for percutaneous absorption is from 60% by mass to 90% by mass, and
wherein a content of a monohydric alcohol having 2 to 4 carbon atoms with respect to the total amount of the composition for percutaneous absorption is equal to or less than 10% by mass.

2. The composition for percutaneous absorption according to claim 1, further comprising water, wherein a content of the water with respect to the total amount of the composition for percutaneous absorption is from 5% by mass to 35% by mass.

3. The composition for percutaneous absorption according to claim 1 or 2, wherein the content of the monohydric alcohol having 2 to 4 carbon atoms with respect to the total amount of the composition for percutaneous absorption is equal to or less than 4% by mass.

4. The composition for percutaneous absorption according to any one of claims 1 to 3, wherein the content of the monohydric alcohol having 2 to 4 carbon atoms with respect to the total amount of the composition for percutaneous absorption is equal to or less than 0.1% by mass.

5. The composition for percutaneous absorption according to any one of claims 1 to 4, wherein a content of the testosterone with respect to the total amount of the composition for percutaneous absorption is from 0.5% by mass to 10% by mass.

6. The composition for percutaneous absorption according to any one of claims 1 to 5, wherein the organic solvents include one or more organic solvents selected from the group consisting of propylene glycol and 1,3-butylene glycol.

7. The composition for percutaneous absorption according to any one of claims 1 to 6, wherein the organic solvents include propylene glycol.

8. The composition for percutaneous absorption according to any one of claims 1 to 7, further comprising a gelling agent.

9. The composition for percutaneous absorption according to any one of claims 1 to 8, wherein a viscosity measured at a shear rate of 100 s⁻¹ and 25°C is within a range of from 0.5 Pa·s to 5 Pa·s.
